# EUROPEAN PATENT APPLICATION

(11) **EP 4 159 116 A1**
(43) Date of publication of application: **05.04.2023**
(21) Application number: 21812668.8
(22) Date of filing: 20.04.2021
(51) Int. Cl.: A61B 5/0205

(54) **TEST METHOD AND APPARATUS**

(30) Priority: 29.05.2020 CN 202010472976
(71) Applicant: BEIJING JINGDONG TUOXIAN TECHNOLOGY CO., LTD., Beijing 100176 (CN)
(72) Inventor: WANG, Qinggang, Beijing 100176 (CN)
(74) Representative: Mewburn Ellis LLP
(86) International application number: PCT/CN2021/088363
(87) International publication number: WO 2021/238503

(57) **Abstract**

A test method and apparatus relating to the technical field of tests. The method comprises: in response to the situation that the two hands of a user to be tested are placed in a preset interaction area of the test apparatus, performing the following test steps: transmitting an electric signal to the two hands, and receiving a feedback signal of the two hands, wherein the feedback signal is used for generating electrical impedance information of the two hands; testing a bioelectric signal of the two hands, wherein the bioelectric signal is used for generating electrocardio information of said user; emitting light having a preset wavelength to the two hands, and receiving reflected light by using a photosensitive sensor in the test apparatus, wherein the reflected light is used for generating blood flow-related index information of said user. According to the method and apparatus, multiple physical indexes of said user can be efficiently and accurately tested by means of one test apparatus; moreover, the multiple physical indexes are obtained by only using the two hands of said user.

## Description

### CROSS-REFERENCE TO RELATED APPLICATIONS

This patent application claims the priority of the Chinese patent application filed on May 29, 2020, with the application number of 202010472976.4 and the invention titled "method and apparatus for testing", the entity of which is incorporated into this application by reference.

### TECHNICAL FIELD

Embodiments of the present disclosure relate to the field of computer technology, in particular to the field of testing technology, and more particularly, to a method and test apparatus.

### BACKGROUND

With the development of technology in the field of medical equipment, more and more people begin to pay attention to their own health. In order to facilitate users to perform health examinations at home and avoid the trouble of queuing in public scenes such as hospitals, various apparatuses for body testing emerge in an endless stream. In order to meet the needs of home use, these apparatuses are often small in size, and the users may use these apparatuses to perform timely testing, so as to conveniently know their own physical conditions.

In the related art, ordinary users may use measuring apparatuses to test routine parameters such as blood pressure and weight by themselves.

### SUMMARY

A method and apparatus for testing, an electronic device and a storage medium are provided.

According to a first aspect, a method for testing is provided, including: performing, in response to a situation that two hands of a to-be-tested user are placed in a preset interaction area of the test apparatus, following test steps: transmitting an electric signal to the two hands, and receiving a feedback signal of the two hands, wherein the feedback signal is used for generating electrical impedance information of the two hands; testing a bioelectric signal of the two hands, wherein the bioelectric signal is used for generating electrocardio information of the to-be-tested user; and emitting light having a preset wavelength to the two hands, and receiving reflected light by using a photosensitive sensor in the test apparatus , wherein the reflected light is used for generating blood flow-related index information of the to-be-tested user.

According to a second aspect, a test apparatus is provided, including a test apparatus, the test apparatus including a control module, a bioimpedance module, an electrocardio module and a pulse module; and the apparatus comprising: the control module, configured to send a test instruction to the bioimpedance module, the electrocardio module and the pulse module, in response to the situation that two hands of a to-be-tested user are placed in a preset interaction area of the test apparatus; the bioimpedance module, configured to transmit an electric signal to the two hands and receive a feedback signal of the two hands, in response to receiving the test instruction, wherein the feedback signal is used for generating electrical impedance information of the two hands; the electrocardio module, configured to test a bioelectric signal of the two hands, in response to receiving the test instruction, wherein the bioelectric signal is used for generating electrocardio information of the to-be-tested user; and the pulse module, configured to emit light having a preset wavelength to the two hands, and use a photosensitive sensor in the pulse module for receiving reflected light, in response to receiving the test instruction, wherein the reflected light is used for generating blood flow-related index information of the to-be-tested user.

According to a third aspect, a test apparatus is provided, the test apparatus including: a testing unit, configured to perform, in response to a situation that two hands of a to-be-tested user are placed in a preset interaction area of the test apparatus, following test steps: transmitting an electric signal to the two hands, and receiving a feedback signal of the two hands, wherein the feedback signal is used for generating electrical impedance information of the two hands; testing a bioelectric signal of the two hands, wherein the bioelectric signal is used for generating electrocardio information of the to-be-tested user; and emitting light having a preset wavelength to the two hands, and receiving reflected light by using a photosensitive sensor in the test apparatus, wherein the reflected light is used for generating blood flow-related index information of the to-be-tested user.

According to a fourth aspect, embodiments of the present disclosure provide an electronic device, comprising: one or more processors; and a memory, storing one or more programs, wherein the one or more programs, when executed by the one or more processors, cause the one or more processors to implement any one of the embodiments of the present disclosure of the method for testing.

According to a fifth aspect, embodiments of the present disclosure provide a computer-readable medium, storing a computer program thereon, wherein the program, when executed by a processor, causes the processor to implement any one of the embodiments of the present disclosure of the method for testing.

According to the solution of the present disclosure, it is possible to efficiently and accurately test a plurality of physical indicators of a to-be-tested user by using an test apparatus. Moreover, the present disclosure may acquire the plurality of physical indicators by only using the two hands of the to-be-tested user.

### BRIEF DESCRIPTION OF THE DRAWINGS

After reading detailed descriptions of non-limiting embodiments with reference to the following accompanying drawings, other features, objectives, and advantages of the present disclosure will become more apparent:
Fig. 1 is an exemplary system architecture diagram to which some embodiments of the present disclosure may be applied;
Fig. 2 is a flowchart of an embodiment of a method for testing according to the present disclosure;
Fig. 3 is a schematic diagram of a preset interaction area of the method for testing according to the present disclosure;
Fig. 4 is a schematic structural diagram of an embodiment of an apparatus for testing according to the present disclosure; and
Fig. 5 is a block diagram of an electronic device used to implement the method for testing according to an embodiment of the present disclosure.

### DETAILED DESCRIPTION OF EMBODIMENTS

Example embodiments of the present disclosure are described below with reference to the accompanying drawings, where various details of the embodiments of the present disclosure are included to facilitate understanding, and should be considered merely as examples. Therefore, those of ordinary skills in the art should realize that various changes and modifications can be made to the embodiments described here without departing from the scope and spirit of the present disclosure. Similarly, for clearness and conciseness, descriptions of well-known functions and structures are omitted in the following description.

It should be noted that the embodiments of the present disclosure and features of the embodiments may be combined with each other on a non-conflict basis. The present disclosure will be described in detail below with reference to the accompanying drawings and in conjunction with the embodiments.

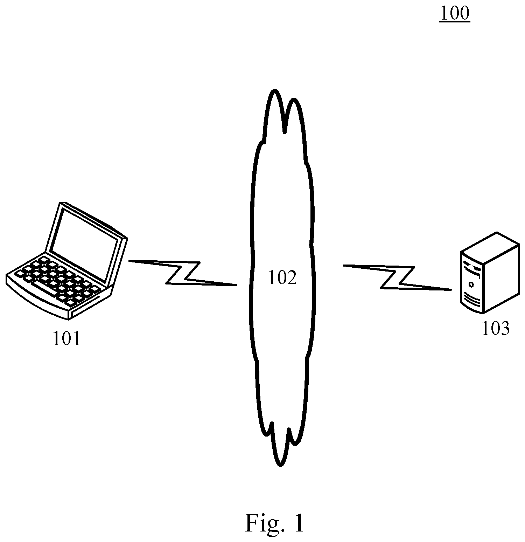

Fig. 1 shows an exemplary system architecture 100 to which embodiments of a method for testing or an apparatus for testing of the present disclosure may be applied.

As shown in Fig. 1, the system architecture 100 may include a terminal device 101, a network 102 and a server 103. The network 102 serves as a medium providing a communication link between the terminal device 101 and the server 103. The network 102 may include various types of connections, such as wired or wireless communication links, or optical cables.

A user may use the terminal device 101 to interact with the server 103 through the network 102 to receive or send messages or the like. Various communication client applications may be installed on the terminal device 101, such as testing parameter display applications, live-stream applications, instant communication tools, email clients, or social platform software.

The terminal device 101 here may be hardware or software. When the terminal device 101 is hardware, it may be various electronic devices having display screens, including but not limited to smart phones, tablet computers, e-book readers, laptop computers, desktop computers, or the like. When the terminal device 101 is software, it may be installed in the electronic devices listed above. It may be implemented as a plurality of software or software modules (e.g., a plurality of software or software modules for providing distributed services), or may be implemented as a single software or software module, which is not limited herein.

The server 103 may be a server that provides various services, such as a backend server that provides support for the terminal device 101. The backend server may process (e.g., analyze) data such as a received feedback signal of an electric signal, a bioelectric signal and reflected light, and feed back a processing result (such as electrical impedance information, electrocardio information and blood flow-related index information) to the terminal device.

It should be noted that the method for testing provided by the embodiments of the present disclosure may be performed by the server 103 or the terminal device 101. Correspondingly, the apparatus for testing may be provided in the server 103 or the terminal device 101.

It should be understood that the numbers of terminal devices, networks, and servers in Fig. 1 are merely illustrative. Any number of terminal devices, networks, and servers may be provided according to implementation needs.

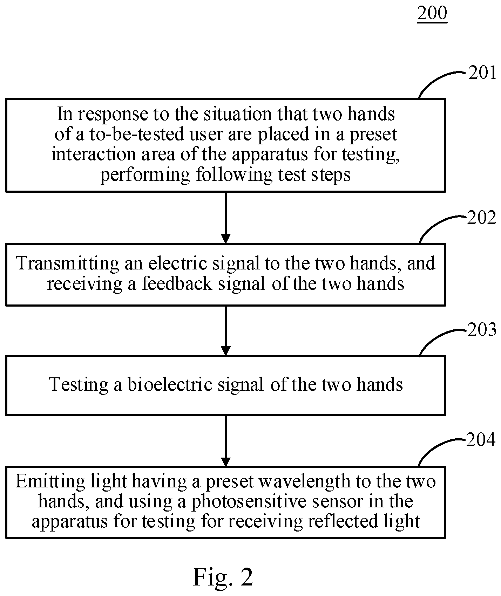

With further reference to Fig. 2, illustrating a flow 200 of an embodiment of a method for testing according to the present disclosure. The method for testing includes the following steps:

Step 201, in response to a situation that two hands of a to-be-tested user are placed in a preset interaction area of the test apparatus, performing following test steps.

In the present embodiment, an executing body (for example, the terminal device shown in Fig. 1) on which the method for testing operates may perform the following test steps 202-204, in response to the situation that the two hands of the to-be-tested user are placed in the preset interaction area of the test apparatus. Here, the to-be-tested user refers to a user to be tested. The preset interaction area refers to an area where the to-be-tested user interacts with the test apparatus. That is, an area where the user places the two hands.

In practice, the executing body may determine that the two hands of the to-be-tested user are placed in the preset interaction area in various ways. For example, the test apparatus may include a gravity sensing component, an infrared sensing component or a thermal sensing component. Once the to-be-tested user places the two hands in the preset interaction area, any of the components can determine that the two hands of the to-be-tested user are placed in the preset interaction area.

Step 202, transmitting an electric signal to the two hands, and receiving a feedback signal of the two hands, where the feedback signal is used for generating electrical impedance information of the two hands.

In the present embodiment, the executing body may transmit the electric signal to the two hands of the to-be-tested user, and receive the feedback signal of the two hands. Then, the executing body may send the feedback signal to the server, so that the server uses the feedback signal to generate the electrical impedance information of the two hands. In addition, the executing body may also directly generate the electrical impedance information locally by using the feedback information without sending. The electric signal may be a voltage signal or a current signal.

In practice, the executing body may perform bioimpedance measurement (BIM) on the two hands, to obtain the feedback signal. In this way, the executing body or other electronic devices may generate a test result, that is, the electrical impedance information. Alternatively, the executing body or other electronic devices may input the electrical impedance information into a preset model to obtain disease information corresponding to the electrical impedance information. Alternatively, the executing body or other electronic devices may look up the disease information corresponding to the electrical impedance information in a correspondence table. The disease information here may include disease names, such as postoperative effusion, pulmonary edema, or anorexia nervosa.

Step 203, testing a bioelectric signal of the two hands, where the bioelectric signal is used for generating electrocardio information of the to-be-tested user.

In the present embodiment, the executing body may test the bioelectric signal of the two hands of the to-be-tested user. In particular, the executing body may acquire the bioelectric signal. In this way, the executing body or other electronic devices may use electrocardiogram (ECG) testing technology to generate a test result based on the bioelectric signal, that is, the electrocardio information of the to-be-tested user. For example, the executing body may send the bioelectric signal to the server, so that the server generates the electrocardio information. In particular, the electrocardio information here refers to an electrocardiogram. The executing body may acquire the bioelectric signal through a lead electrode set.

Alternatively, the executing body may display and prompt the user to wear a plurality of apparatuses provided with lead electrode sets on multiple parts of the body, such as wrists and ankles. The executing body or other electronic devices may generate the electrocardio information by using the bioelectric signal acquired by each electrode set.

Step 204, emitting light having a preset wavelength to the two hands, and receiving reflected light by using a photosensitive sensor in the test apparatus for, where the reflected light is used for generating blood flow-related index information of the to-be-tested user.

In the present embodiment, the test apparatus includes the photosensitive sensor. The executing body may emit the light having the preset wavelength (such as LED light) to the two hands, and use the photosensitive sensor for receiving the reflected light of the light. Then, the executing body or other electronic devices may use the reflected light to generate the blood flow-related index information of the to-be-tested user. For example, the executing body may upload the reflected light to the server, so that the server determines the blood flow-related index information based on the reflected light. In particular, the blood flow-related index information refers to information used to represent blood flow characteristics, for example, the blood flow-related index information may include at least one of the following: blood oxygen, blood pressure, or heart rate.

In practice, the executing body or other electronic devices may use photo plethysmo graphy (PPG) to convert the reflected light into an electric signal, and extract an AC signal in the electric signal and convert the AC signal into a digital signal as a result of the PPG, that is, the blood flow-related index information.

According to the method provided by the above embodiment of the present disclosure, it is possible to efficiently and accurately test a plurality of physical indicators of a to-be-tested user by using an test apparatus. Moreover, the present disclosure may acquire the plurality of physical indicators by only using the two hands of the to-be-tested user.

In some alternative implementations of the present embodiment, the preset interaction area may include a first sub-area; and the transmitting an electric signal to the two hands, and receiving a feedback signal of the two hands in step 202, may include: transmitting the electric signal to the two hands and receiving the feedback signal of the two hands in the first sub-area, where the number of the first sub-areas is at least two, and at least two of the first sub-areas respectively correspond to the two hands.

In these alternative implementations, the preset interaction area may include a plurality of sub-areas. In particular, the preset interaction area may include the first sub-area. The executing body may transmit the electric signal to the two hands of the to-be-tested user, and receive the feedback signal in the first sub-area. The executing body may transmit the electric signal within a range of the first sub-area in a preset transmitting direction of the electric signal, so as to locally transmit the electric signal to the two hands in the first sub-area.

The number of the first sub-areas is at least two, for example, it may be four. At least two of the first sub-areas respectively correspond to each of the two hands, that is, in the at least two first sub-areas, at least one first sub-area corresponds to one of the two hands, and the other first sub-area other than the at least one first sub-area corresponds to the other hand.

These implementations may realize testing of the electrical impedance information in a dedicated sub-area where the electric signal is transmitted and the feedback signal is received, which helps to improve test accuracy.

In some alternative application scenarios of these implementations, the test apparatus is provided with a plurality of first electrodes, and in the plurality of first electrodes, a first electrode corresponding to a left hand and a first electrode corresponding to a right hand are symmetrically arranged; and the transmitting the electric signal to the two hands and receiving the feedback signal of the two hands in the first sub-area in these implementations, may include: transmitting the electric signal to the two hands and receiving the feedback signal of the two hands in the first sub-area, using the plurality of first electrodes.

In these alternative application scenarios, the test apparatus is provided with the plurality of first electrodes, and these first electrodes may be symmetrically arranged. For example, there are four first electrodes in total, and the positions of two first electrodes arranged on the left side (i.e., arranged for the left hand) and the positions of two first electrodes arranged on the right side (i.e., arranged for the right hand) are symmetrical. The executing body may use the first electrodes to transmit the electric signal. The number of the first electrodes corresponding to the left hand and the number of the first electrodes corresponding to the right hand are equal and the electrodes are symmetrically arranged.

These application scenarios may use the symmetrical electrodes for testing to reduce test deviation caused by contact impedance between the two hands and the electrodes.

In some alternative implementations of the present embodiment, the preset interaction area includes a second sub-area, and the test apparatus is provided with a plurality of second electrodes; and the testing a bioelectric signal of the two hands in step 203, may include: testing the bioelectric signal of the two hands in the second sub-area using the plurality of second electrodes, where the number of the second sub-areas is at least two, at least two of the second sub-areas respectively correspond to the two hands, the second electrode is an electrode sheet, an area of the electrode sheet is greater than a specified area threshold, and in at least two of the electrode sheets, an electrode sheet corresponding to the left hand and an electrode sheet corresponding to the right hand are symmetrically arranged.

In these alternative implementations, the executing body may test the bioelectric signal of the two hands in the second sub-area using the plurality of second electrodes. In particular, the second electrode may be an electrode sheet having a large area. The number of the electrode sheets corresponding to the left hand and the number of the electrode sheets corresponding to the right hand are equal and the electrode sheets are symmetrically arranged.

These implementations may perform testing in a dedicated sub-area for testing the bioelectric signal, which helps to improve the test accuracy. Moreover, these implementations may use large electrode sheets for testing, which solves the problem that the bioelectric signal is difficult to capture, and by using the symmetrical electrode sheets, deviation in test results is reduced.

In some alternative implementations of the present embodiment, the preset interaction area includes a third sub-area; and the emitting light having a preset wavelength to the two hands, and receiving reflected light by using a photosensitive sensor in the test apparatus in step 204, may include: emitting the light having the preset wavelength to the two hands, and using the photosensitive sensor in the test apparatus for receiving the reflected light in the third sub-area, where the number of the third sub-areas is at least one.

In these alternative implementations, the executing body may emit the light having the preset wavelength in the third sub-area, and receive the reflected light in the third sub-area. That is, the executing body may emit light to at least one hand of the two hands, and receive reflected light of the at least one hand.

These implementations may perform testing in a dedicated sub-area where light is emitted and received, which helps to improve test accuracy on the blood flow-related index information.

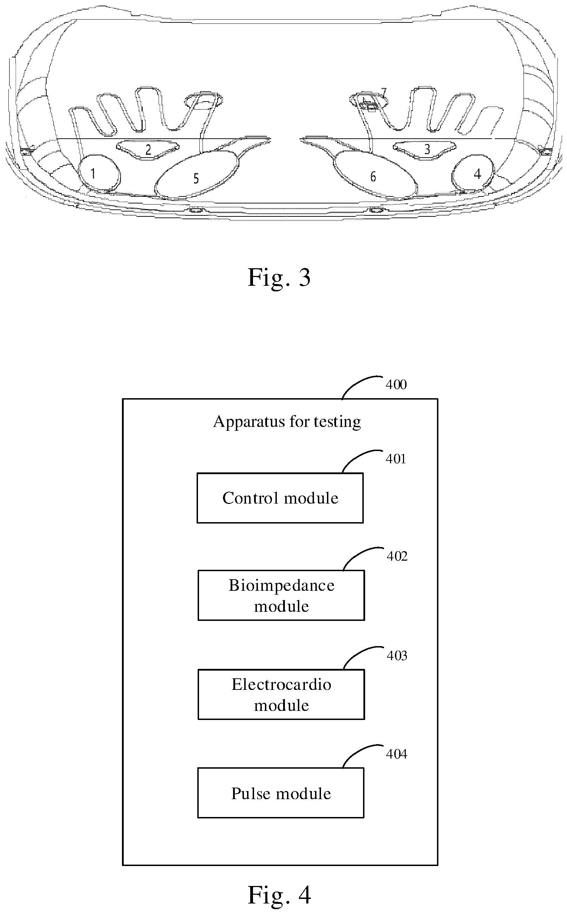

With further reference to Fig. 3, Fig. 3 is a schematic diagram of a preset interaction area of the method for testing according to the present embodiment. In the schematic diagram, the preset interaction area is set as a hand-shaped frame. The first sub-areas 1, 2 and 3, 4 in the figure include measurement electrodes of the BIM. The second sub-areas 5 and 6 include measurement electrodes of the ECG. The third sub-area 7 is a measurement area of the PPG.

By arranging the four measurement electrodes for BIM testing symmetrically and the two measurement electrodes for BCG testing symmetrically, the test accuracy may be improved, and environmental interference and contact error may be reduced. For example, when performing BIM testing on two hands, the above four measurement electrodes are symmetrically arranged for measurement. After the two hands touch the electrodes, the human body and a BIM measurement module form a closed-loop system, and an electrode system emits a tiny AC measurement current or voltage to the body tissue to test impedance of the human body. Because of the symmetrical design of the electrodes, after the two hands of the human body touch the electrodes, a symmetrical position is maintained, so a measurement error caused by the contact impedance between the two hands and the electrode sheets may be offset. At the same time, the symmetrical design may also offset a measurement error caused by basic impedance between the two hands and the electrode sheets. Here, for ECG testing, symmetrical large electrode sheets are used, so that more people can be taken care of, and there will be no measurement errors caused by too large or too small of to-be-tested two hands. Here, for PPG measurement in Fig. 3, it is placed on the index finger of the right hand. PPG signal measurement is placed on the finger, which does not interfere with ECG measurement and BIM measurement, is simple and convenient. In some alternative implementations, a PPG measurement area may also be set in areas corresponding to other fingers.

With further reference to Fig. 4, illustrating a schematic structural diagram of an embodiment of an test apparatus. The test apparatus may include a control module, a bioimpedance module, an electrocardio module and a pulse module. The apparatus may include: the control module, configured to send a test instruction to the bioimpedance module, the electrocardio module and the pulse module, in response to the situation that two hands of a to-be-tested user are placed in a preset interaction area of the test apparatus; the bioimpedance module, configured to transmit an electric signal to the two hands and receive a feedback signal of the two hands, in response to receiving the test instruction, where the feedback signal is used for generating electrical impedance information of the two hands; the electrocardio module, configured to test a bioelectric signal of the two hands, in response to receiving the test instruction, where the bioelectric signal is used for generating electrocardio information of the to-be-tested user; and the pulse module, configured to emit light having a preset wavelength to the two hands, and use a photosensitive sensor in the pulse module for receiving reflected light, in response to receiving the test instruction, where the reflected light is used for generating blood flow-related index information of the to-be-tested user.

According to the apparatus provided by the above embodiment of the present disclosure, it is possible to efficiently and accurately test a plurality of physical indicators of a to-be-tested user by using an test apparatus. Moreover, the present disclosure may acquire the plurality of physical indicators by only using the two hands of the to-be-tested user.

Alternatively, the preset interaction area includes a first sub-area, a second sub-area and a third sub-area respectively used for testing of the bioimpedance module, the electrocardio module and the pulse module.

Alternatively, the preset interaction area includes a first sub-area; and the bioimpedance module is further configured to transmit the electric signal to the two hands and receive the feedback signal of the two hands in the first sub-area, where the number of the first sub-areas is at least two, and at least two of the first sub-areas respectively correspond to the two hands.

Alternatively, the bioimpedance module is provided with a plurality of first electrodes, and in the plurality of first electrodes, a first electrode corresponding to a left hand and a first electrode corresponding to a right hand are symmetrically arranged; and the bioimpedance module is further configured to transmit the electric signal to the two hands and receive the feedback signal of the two hands in the first sub-area, using the plurality of first electrodes.

Alternatively, the preset interaction area includes the second sub-area, and the electrocardio module is provided with a plurality of second electrodes; and the electrocardio module is further configured to test the bioelectric signal of the two hands in the second sub-area using the plurality of second electrodes, where the number of the second sub-areas is at least two, at least two of the second sub-areas respectively correspond to the two hands, the second electrode is an electrode sheet, an area of the electrode sheet is greater than a specified area threshold, and in at least two of the electrode sheets, an electrode sheet corresponding to the left hand and an electrode sheet corresponding to the right hand are symmetrically arranged.

Alternatively, the preset interaction area includes the third sub-area; and the pulse module is further configured to emit the light having the preset wavelength to the two hands, and use the photosensitive sensor in the test apparatus for receiving the reflected light in the third sub-area, where the number of the third sub-areas is at least one.

As an implementation of the method shown in the above figures, the present disclosure provides an embodiment of an test apparatus, which corresponds to the method embodiment shown in Fig. 2. In addition to the features described below, the apparatus embodiment may also include the same or corresponding features or effects as the method embodiment shown in Fig. 2. The apparatus may be applied to various electronic devices.

The test apparatus of the present embodiment includes a testing unit. The testing unit is configured to, in response to the situation that two hands of a to-be-tested user are placed in a preset interaction area of the test apparatus, perform following test steps: transmitting an electric signal to the two hands, and receiving a feedback signal of the two hands, where the feedback signal is used for generating electrical impedance information of the two hands; testing a bioelectric signal of the two hands, where the bioelectric signal is used for generating electrocardio information of the to-be-tested user; and emitting light having a preset wavelength to the two hands, and receiving reflected light by using a photosensitive sensor in the test apparatus, where the reflected light is used for generating blood flow-related index information of the to-be-tested user.

In the present embodiment, for the specific processing and the technical effects of the test apparatus, reference may be made to the relevant description of step 201, step 202, step 203 and step 204 in the corresponding embodiment of Fig. 2, and detailed description thereof will be omitted.

In some alternative implementations of the present embodiment, the preset interaction area includes a first sub-area; and the transmitting an electric signal to the two hands, and receiving a feedback signal of the two hands, includes: transmitting the electric signal to the two hands and receiving the feedback signal of the two hands in the first sub-area, where the number of the first sub-areas is at least two, and at least two of the first sub-areas respectively correspond to the two hands.

In some alternative implementations of the present embodiment, the test apparatus is provided with a plurality of first electrodes, and in the plurality of first electrodes, a first electrode corresponding to a left hand and a first electrode corresponding to a right hand are symmetrically arranged; and the transmitting the electric signal to the two hands and receiving the feedback signal of the two hands in the first sub-area, includes: transmitting the electric signal to the two hands and receiving the feedback signal of the two hands in the first sub-area, using the plurality of first electrodes.

In some alternative implementations of the present embodiment, the preset interaction area includes a second sub-area, and the test apparatus is provided with a plurality of second electrodes; and the testing a bioelectric signal of the two hands, includes: testing the bioelectric signal of the two hands in the second sub-area using the plurality of second electrodes, where the number of the second sub-areas is at least two, at least two of the second sub-areas respectively correspond to the two hands, the second electrode is an electrode sheet, an area of the electrode sheet is greater than a specified area threshold, and in at least two of the electrode sheets, an electrode sheet corresponding to the left hand and an electrode sheet corresponding to the right hand are symmetrically arranged.

In some alternative implementations of the present embodiment, the preset interaction area includes a third sub-area; and the emitting light having a preset wavelength to the two hands, and receiving reflected light by using a photosensitive sensor in the test apparatus , includes: emitting the light having the preset wavelength to the two hands, and using the photosensitive sensor in the test apparatus for receiving the reflected light in the third sub-area, where the number of the third sub-areas is at least one.

According to an embodiment of the present disclosure, the present disclosure also provides an electronic device and a readable storage medium.

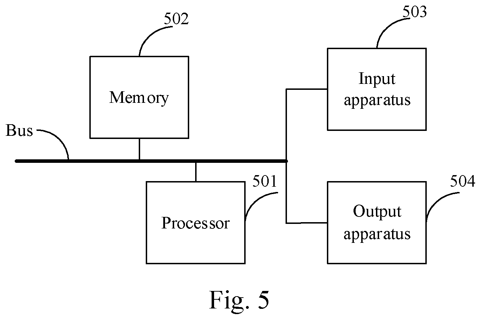

As shown in Fig. 5, is a block diagram of an electronic device of the method for testing according to an embodiment of the present disclosure. The electronic device is intended to represent various forms of digital computers, such as laptop computers, desktop computers, workbenches, personal digital assistants, servers, blade servers, mainframe computers, and other suitable computers. The electronic device may also represent various forms of mobile apparatuses, such as personal digital processors, cellular phones, smart phones, wearable devices, and other similar computing apparatuses. The components shown herein, their connections and relationships, and their functions are merely examples, and are not intended to limit the implementation of the present disclosure described and/or claimed herein.

As shown in Fig. 5, the electronic device includes: one or more processors 501, a memory 502, and interfaces for connecting various components, including high-speed interfaces and low-speed interfaces. The various components are interconnected using different buses and may be mounted on a common motherboard or otherwise as desired. The processor may process instructions executed within the electronic device, including instructions stored in or on the memory to display graphical information of a GUI on an external input/output apparatus, such as a display device coupled to the interface. In other embodiments, a plurality of processors and/or a plurality of buses may be used with a plurality of memories and a plurality of memories, if desired. Likewise, a plurality of electronic devices may be connected, each providing some of the necessary operations (e.g., as a server array, a set of blade servers, or a multiprocessor system). One processor 501 is used as an example in Fig. 5.

The memory 502 is a non-transitory computer readable storage medium provided by the present disclosure. The memory stores instructions executable by at least one processor, so that the at least one processor executes the method for testing provided by the present disclosure. The non-transitory computer readable storage medium of the present disclosure stores computer instructions, and the computer instructions are used to cause the computer to perform the method for testing provided by the present disclosure.

As a non-transitory computer readable storage medium, the memory 502 may be used to store non-transitory software programs, non-transitory computer-executable programs, and modules, such as program instructions/modules (for example, the testing unit) corresponding to the method for testing in the embodiments of the present disclosure. The processor 501 executes various functional applications and data processing of the server by running the non-transitory software programs, instructions and modules stored in the memory 502, that is, implements the method for testing in the above method embodiments.

The memory 502 may include a stored program area and a stored data area, where the stored program area may store an operating system, an application program required by at least one function; and the stored data area may store data created according to the use of the electronic device for testing, etc. Additionally, the memory 502 may include a high-speed random access memory, and may also include a non-transitory memory, such as at least one magnetic disk storage device, a flash memory device, or other non-transitory solid-state storage device. In some embodiments, the memory 502 may optionally include memories located remotely from the processor 501, and these remote memories may be connected to the electronic device for testing via a network. Examples of such network include, but are not limited to, the Internet, an intranet, a local area network, a mobile communication network, and combinations thereof.

The electronic device of the method for testing may further include: an input apparatus 503 and an output apparatus 504. The processor 501, the memory 502, the input apparatus 503 and the output apparatus 504 may be connected via a bus or in other ways, and the connection via a bus is used as an example in Fig. 5.

The input apparatus 503 may receive input digital or character information, and generate key signal inputs related to user settings and function control of the electronic device of the method for testing, such as touch screen, keypad, mouse, trackpad, touchpad, pointing stick, one or more mouse buttons, trackball, joystick and other input apparatuses. The output apparatus 504 may include a display device, an auxiliary lighting apparatus (for example, LED), a tactile feedback apparatus (for example, a vibration motor), and the like. The display device may include, but is not limited to, a liquid crystal display (LCD), a light emitting diode (LED) display, and a plasma display. In some embodiments, the display device may be a touch screen.

Various embodiments of the systems and technologies described herein may be implemented in digital electronic circuit systems, integrated circuit systems, dedicated ASICs (application specific integrated circuits), computer hardware, firmware, software, and/or combinations thereof. These various embodiments may include: being implemented in one or more computer programs that can be executed and/or interpreted on a programmable system that includes at least one programmable processor. The programmable processor may be a dedicated or general-purpose programmable processor, and may receive data and instructions from a storage system, at least one input apparatus, and at least one output apparatus, and transmit the data and instructions to the storage system, the at least one input apparatus, and the at least one output apparatus.

These computing programs (also referred to as programs, software, software applications, or codes) include machine instructions of the programmable processor and may use high-level processes and/or object-oriented programming languages, and/or assembly/machine languages to implement these computing programs. As used herein, the terms "machine readable medium" and "computer readable medium" refer to any computer program product, device, and/or apparatus (for example, magnetic disk, optical disk, memory, programmable logic apparatus (PLD)) used to provide machine instructions and/or data to the programmable processor, including machine readable medium that receives machine instructions as machine readable signals. The term "machine readable signal" refers to any signal used to provide machine instructions and/or data to the programmable processor.

In order to provide interaction with a user, the systems and technologies described herein may be implemented on a computer, the computer has: a display apparatus for displaying information to the user (for example, CRT (cathode ray tube) or LCD (liquid crystal display) monitor); and a keyboard and a pointing apparatus (for example, mouse or trackball), and the user may use the keyboard and the pointing apparatus to provide input to the computer. Other types of apparatuses may also be used to provide interaction with the user; for example, feedback provided to the user may be any form of sensory feedback (for example, visual feedback, auditory feedback, or tactile feedback); and any form (including acoustic input, voice input, or tactile input) may be used to receive input from the user.

The systems and technologies described herein may be implemented in a computing system that includes backend components (e.g., as a data server), or a computing system that includes middleware components (e.g., application server), or a computing system that includes frontend components (for example, a user computer having a graphical user interface or a web browser, through which the user may interact with the implementations of the systems and the technologies described herein), or a computing system that includes any combination of such backend components, middleware components, or frontend components. The components of the system may be interconnected by any form or medium of digital data communication (e.g., communication network). Examples of the communication network include: local area networks (LAN), wide area networks (WAN), the Internet, and blockchain networks.

The computer system may include a client and a server. The client and the server are generally far from each other and usually interact through the communication network. The relationship between the client and the server is generated by computer programs that run on the corresponding computer and have a client-server relationship with each other.

The flow charts and block diagrams in the attached drawings illustrate the architecture, functions and operations of possible implementations of systems, methods and computer program products in accordance with the various embodiments of the application. At this point, each box in a flowchart or block diagram can represent a module, program segment, or part of code that contains one or more executable instructions for implementing a specified logical function. It should also be noted that in some implementations of substitution, the functions indicated in the box may also occur in a different order than those indicated in the accompanying figure. For example, two boxes that are represented consecutively can actually be executed almost in parallel, or they can sometimes be executed in reverse order, depending on the functionality involved. It should also be noted that each box in a block diagram and/or flowchart, and a combination of boxes in a block diagram and/or flowchart, may be implemented using a dedicated hardware-based system that performs a specified function or operation, or may be implemented using a combination of dedicated hardware and computer instructions.

The units involved in the embodiments of the present disclosure may be implemented by means of software or hardware. The described units may also be provided in a processor, for example, may be described as: a processor including a testing unit. Here, the names of these units do not in some cases constitute limitations to such units themselves.

As another aspect, the present disclosure also provides a computer readable medium, where the computer readable medium may be included in the apparatus described in the foregoing embodiment, or a stand-alone computer readable medium not assembled into the apparatus. The computer readable medium carries one or more programs. The one or more programs, when executed by the apparatus, cause the apparatus to: in response to the situation that two hands of a to-be-tested user are placed in a preset interaction area of the test apparatus, perform following test steps: transmitting an electric signal to the two hands, and receiving a feedback signal of the two hands, where the feedback signal is used for generating electrical impedance information of the two hands; testing a bioelectric signal of the two hands, where the bioelectric signal is used for generating electrocardio information of the to-be-tested user; and emitting light having a preset wavelength to the two hands, and receiving reflected light by using a photosensitive sensor in the test apparatus , where the reflected light is used for generating blood flow-related index information of the to-be-tested user.

The above description is only a better embodiment of the present application and an explanation of the technical principles used. The technical personnel in this field shall understand that the scope of invention covered in this application is not limited to technical solutions resulting from a particular combination of the above technical characteristics, but shall also cover other technical solutions resulting from any combination of the above technical characteristics or their equivalent without leaving the invention concept. For example(but not limited to), the technical solution formed by substituting the above features with the technical features with similar functions disclosed in this application.

## Claims

1. A method for testing a test apparatus, the method comprising:
performing, in response to a situation that two hands of a to-be-tested user are placed in a preset interaction area of the test apparatus, following test steps:
transmitting an electric signal to the two hands, and receiving a feedback signal of the two hands, wherein the feedback signal is used for generating electrical impedance information of the two hands;
testing a bioelectric signal of the two hands, wherein the bioelectric signal is used for generating electrocardio information of the to-be-tested user; and
emitting light having a preset wavelength to the two hands, and receiving reflected light by using a photosensitive sensor in the test apparatus , wherein the reflected light is used for generating blood flow-related index information of the to-be-tested user.

2. The method according to claim 1, wherein the preset interaction area comprises a first sub-area; and
the transmitting an electric signal to the two hands, and receiving a feedback signal of the two hands, comprises:
transmitting the electric signal to the two hands and receiving the feedback signal of the two hands in the first sub-area, wherein a number of the first sub-areas is at least two, and at least two of the first sub-areas respectively correspond to the two hands.

3. The method according to claim 2, wherein the test apparatus is provided with a plurality of first electrodes, and in the plurality of first electrodes, a first electrode corresponding to a left hand and a first electrode corresponding to a right hand are symmetrically arranged; and
the transmitting the electric signal to the two hands and receiving the feedback signal of the two hands in the first sub-area, comprises:
transmitting the electric signal to the two hands and receiving the feedback signal of the two hands in the first sub-area, using the plurality of first electrodes.

4. The method according to any one of claims 1-3, wherein the preset interaction area comprises a second sub-area, and the test apparatus is provided with a plurality of second electrodes; and
the testing a bioelectric signal of the two hands, comprises:
testing the bioelectric signal of the two hands in the second sub-area using the plurality of second electrodes, wherein a number of the second sub-areas is at least two, at least two of the second sub-areas respectively correspond to the two hands, the second electrode is an electrode sheet, an area of the electrode sheet is greater than a specified area threshold, and in at least two of the electrode sheets, an electrode sheet corresponding to the left hand and an electrode sheet corresponding to the right hand are symmetrically arranged.

5. The method according to any one of claims 1-4, wherein the preset interaction area comprises a third sub-area; and
the emitting light having a preset wavelength to the two hands, and receiving reflected light by using a photosensitive sensor in the test apparatus, comprises:
emitting the light having the preset wavelength to the two hands, and receiving reflected light by using the photosensitive sensor in the test apparatus in the third sub-area, wherein a number of the third sub-areas is at least one.

6. An test apparatus, the test apparatus comprising a control module, a bioimpedance module, an electrocardio module and a pulse module; and the apparatus comprising:
the control module, configured to send a test instruction to the bioimpedance module, the electrocardio module and the pulse module, in response to the situation that two hands of a to-be-tested user are placed in a preset interaction area of the test apparatus;
the bioimpedance module, configured to transmit an electric signal to the two hands and receive a feedback signal of the two hands, in response to receiving the test instruction, wherein the feedback signal is used for generating electrical impedance information of the two hands;
the electrocardio module, configured to test a bioelectric signal of the two hands, in response to receiving the test instruction, wherein the bioelectric signal is used for generating electrocardio information of the to-be-tested user; and
the pulse module, configured to emit light having a preset wavelength to the two hands, and use a photosensitive sensor in the pulse module for receiving reflected light, in response to receiving the test instruction, wherein the reflected light is used for generating blood flow-related index information of the to-be-tested user.

7. The apparatus according to claim 6, wherein the preset interaction area comprises a first sub-area, a second sub-area and a third sub-area respectively used for testing of the bioimpedance module, the electrocardio module and the pulse module.

8. The apparatus according to claim 6, wherein the preset interaction area comprises a first sub-area; and the bioimpedance module is further configured to transmit the electric signal to the two hands and receive the feedback signal of the two hands in the first sub-area, wherein a number of the first sub-areas is at least two, and at least two of the first sub-areas respectively correspond to the two hands.

9. The apparatus according to claim 8, wherein the bioimpedance module is provided with a plurality of first electrodes, and in the plurality of first electrodes, a first electrode corresponding to a left hand and a first electrode corresponding to a right hand are symmetrically arranged; and the bioimpedance module is further configured to transmit the electric signal to the two hands and receive the feedback signal of the two hands in the first sub-area, using the plurality of first electrodes.

10. The apparatus according to any one of claims 6-9, wherein the preset interaction area comprises a second sub-area, and the electrocardio module is provided with a plurality of second electrodes; and the electrocardio module is further configured to test the bioelectric signal of the two hands in the second sub-area using the plurality of second electrodes, wherein a number of the second sub-areas is at least two, at least two of the second sub-areas respectively correspond to the two hands, the second electrode is an electrode sheet, an area of the electrode sheet is greater than a specified area threshold, and in at least two of the electrode sheets, an electrode sheet corresponding to the left hand and an electrode sheet corresponding to the right hand are symmetrically arranged.

11. The apparatus according to any one of claims 6-10, wherein the preset interaction area comprises a third sub-area; and the pulse module is further configured to emit the light having the preset wavelength to the two hands, and use the photosensitive sensor in the test apparatus for receiving the reflected light in the third sub-area, wherein a number of the third sub-areas is at least one.

12. An test apparatus, the apparatus comprising:
a testing unit, configured to perform, in response to a situation that two hands of a to-be-tested user are placed in a preset interaction area of the test apparatus, following test steps:
transmitting an electric signal to the two hands, and receiving a feedback signal of the two hands, wherein the feedback signal is used for generating electrical impedance information of the two hands;
testing a bioelectric signal of the two hands, wherein the bioelectric signal is used for generating electrocardio information of the to-be-tested user; and
emitting light having a preset wavelength to the two hands, and receiving reflected light by using a photosensitive sensor in the test apparatus, wherein the reflected light is used for generating blood flow-related index information of the to-be-tested user.

13. An electronic device, comprising:
one or more processors;
a storage apparatus, for storing one or more programs; and
the one or more programs, when executed by the one or more processors, cause the one or more processors to implement the method according to any one of claims 1-5.

14. A computer readable storage medium, storing a computer program thereon, wherein, the program, when executed by a processor, implements the method according to any one of claims 1-5.
